# EUROPEAN PATENT APPLICATION

(11) **EP 2 517 746 A1**
(43) Date of publication of application: **31.10.2012**
(21) Application number: 11164285.6
(22) Date of filing: 29.04.2011
(51) Int. Cl.: A61M 5/32

(54) **Needle assembly storage system**

(71) Applicant: Sanofi-Aventis Deutschland GmbH, 65929 Frankfurt (DE)
(72) Inventor: The designation of the inventor has not yet been filed

(57) **Abstract**

Described is a needle assembly storage system (1) comprising a needle assembly (2) including at least one hook (5.4) formed on an outer surface (5.1), and a needle assembly storage device (3) having at least one slot for receiving the at least one hook (5.4). The at least one slot includes a first recess (8.3) formed in a wall (8.1) of the storage device (3). The first recess (8.3) includes a first abutment surface (8.8) and a second abutment surface (8.9) for preventing the at least one hook (5.4) from moving out of the first recess (8.3).

## Description

### Technical Field

The invention relates to a needle assembly storage system which prevents re-use of a needle assembly.

### Background of the Invention

Patients suffering from diseases like diabetes have to frequently self-administer injections. Injection devices like auto-injectors or pen injectors have been developed to facilitate self-administering injections. Typically, such injection devices are re-usable and refitted with sterile, single-use injection needle assemblies to minimize the risk of infections.

There is a risk of needle stick injury if the user if required to mount and dismount from the injection device and/or handle the needle assembly. Thus, attempts, such as that discussed in US 5971966, have been made to overcome this risk. However, there remains a need for devices to prevent needle stick injuries, while minimizing risk of infections and maintaining sterility of the needle assemblies.

### Summary of the Invention

It is an object of the present invention to provide a needle assembly storage system which prevents reuse of a needle assembly.

In an exemplary embodiment, a needle assembly storage system comprises a needle assembly including at least one hook formed on an outer surface, and a needle assembly storage device having at least one slot for receiving the at least one hook. The at least one slot includes a first recess formed in a wall of the storage device. The first recess includes a first abutment surface and a second abutment surface for preventing the at least one hook from moving out of the first recess. The at least one hook may have a first state biased in a position extending beyond a circumference of the outer surface and a second state in alignment with the circumference of the outer surface.

In an exemplary embodiment, an audible feedback is provided when the at least one hook engages the first recess.

The at least one slot may be formed on a bushing removably coupled to the needle assembly storage device.

In an exemplary embodiment, the at least one slot further includes a second recess formed in the wall adjacent the first recess, and a first depth of the first recess in the wall is greater than a second depth of the second recess. The first recess may be coupled to the second recess via a first interface, and the first interface may be an angled surface connecting the first abutment surface to the second depth. The at least one slot may further include a third recess formed in the wall in substantial alignment with the second recess. A third depth of the third recess in the wall may be greater than the second depth of the second recess. The third recess may be coupled to the second recess via a second interface. The second interface may be an angled surface connecting the second depth to the third depth. The third recess may include a proximal end having a width greater than a width of one of the at least one hook.

When the at least one hook is in the first recess, the first abutment surface prevents rotation of the at least one hook in a first rotational direction A and the second abutment surface prevents rotation of the at least one hook in a second rotational direction B.

In an exemplary embodiment, the first recess includes a proximal wall to abut the at least one hook and prevent proximal displacement of the needle assembly relative to the needle assembly storage device.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: shows a sectional view of a needle assembly storage system according to an exemplary embodiment of the present invention,
- Figure 2: shows a sectional view of a needle assembly according to an exemplary embodiment of the present invention,
- Figure 3: shows a sectional view of a housing of a needle assembly storage device according to an exemplary embodiment of the present invention,
- Figure 4: shows a sectional view of a bushing according to an exemplary embodiment of the present invention,
- Figure 5: shows a cut-out of a perspective view of a bushing or surface of a needle assembly storage device according to an exemplary embodiment of the present invention,
- Figure 6: shows a sectional view of a needle assembly storage system according to an exemplary embodiment of the present invention,
- Figure 7: shows a sectional view of a needle assembly according to an exemplary embodiment of the present invention, and

- Figure 8: shows a cross section of a bushing or a needle assembly storage device according to an exemplary embodiment of the present invention.

Corresponding parts are marked with the same reference symbols in all figures.

### Detailed Description

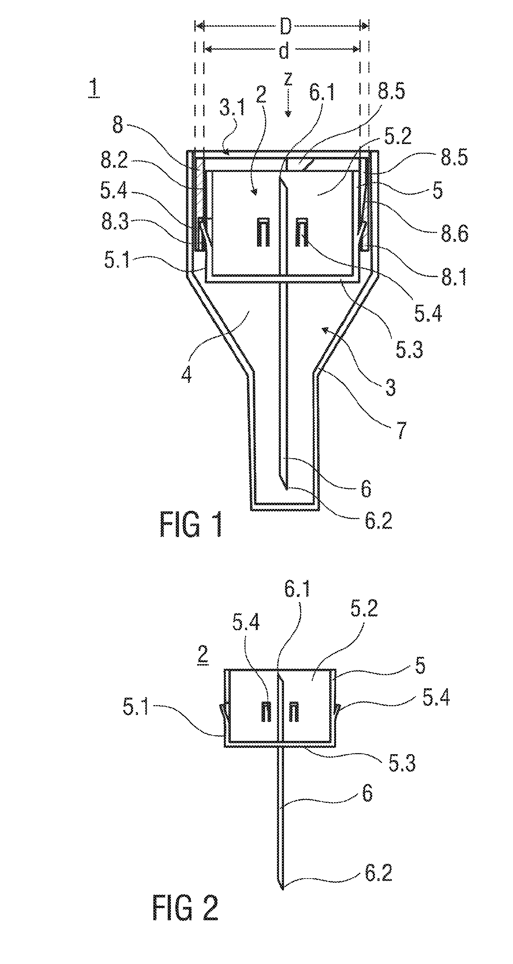

Figures 1 to 5 show an exemplary embodiment of a needle assembly storage system 1 comprising a needle assembly 2 and a needle assembly storage device 3 according to the invention. The storage device 3 includes a cavity 4 which is sized and shaped to receive the needle assembly 2.

Figure 2 shows an exemplary embodiment of the needle assembly 2 according to the present invention. The needle assembly 2 comprises a needle support 5 and a needle 6. In an exemplary embodiment, the needle support 5 is substantially cylindrical having an outer surface 5.1, an opening 5.2 formed on a proximal end, and a cover 5.3 formed on its distal end. The needle support 5 is constructed to be mountable on an injection device (e.g., pen injector, autoinjector, etc.). For example, an inner surface of the needle assembly 5 may include an internal screw thread (not shown in the drawings) for engaging a corresponding external screw thread on the injection device. Those of skill in the art will understand that various coupling mechanisms, other than screw threads, may be used, e.g., bayonet coupling, snap fit, friction fit, etc.

The needle 6 may be mounted in a center of the cover 5.3 and be oriented along a longitudinal axis of the needle support 5. The needle 6 may be double-sided, such that a first needle tip 6.1 extends proximally of the cover 5.3 for penetrating a cartridge septum in the injection device and a second needle tip 6.2 extends distally of the cover 5.3 for penetrating an injection site.

In an exemplary embodiment, the needle support 5 includes one or more retention hooks 5.4 formed on the outer surface 5.1 thereof. A given hook 5.4 may be formed as a projection which extends radially from the outer surface 5.1 and resides in a slot formed in the outer surface 5.1. A distal portion of the hook 5.4 may be connected to the outer surface 5.1, and a proximal portion of the hook 5.4 may be biased in a first state (an extended state) beyond a circumference of the outer surface 5.1. The proximal portion of the hook 5.4 may be positioned in a second state, aligned with the outer surface 5.1, when an external force presses the proximal portion radially inward. In an exemplary embodiment, there are a plurality of the hooks 5.4 which are equally-spaced on the outer surface 5.1.

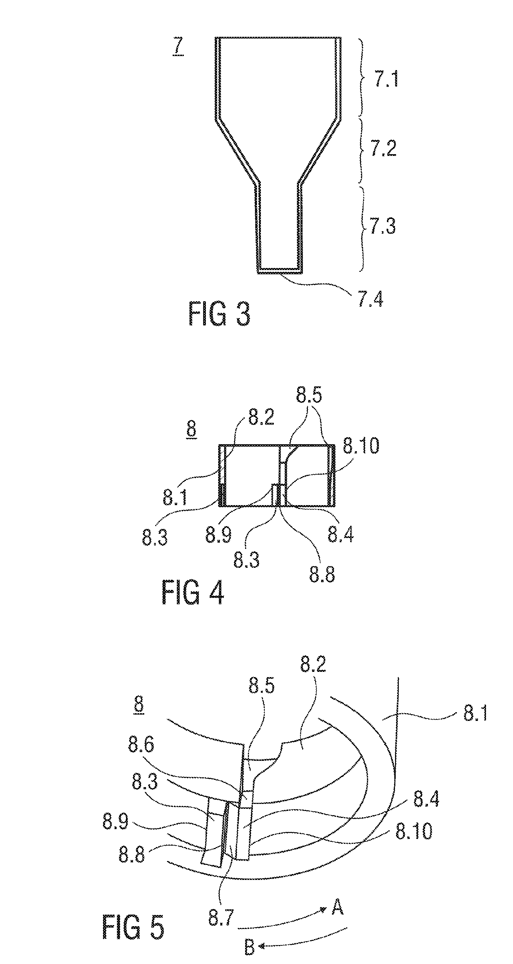

Figure 3 shows a sectional view of an exemplary embodiment of the storage device 3 which comprises a housing 7. The housing 7 comprises a cylindrical proximal portion 7.1 which is sized and shaped to accommodate the needle support 5, a frusto-conical shaped middle portion 7.2 having a diameter which decreases from its proximal to distal ends, and a cylindrical distal portion 7.3 which is sized and shaped to accommodate the needle 6. A distal end 7.4 of the distal portion 7.3 may be closed to prevent exposure and maintain sterility of the needle 6. A proximal end of the housing may include an opening 3.1 for removing an unused needle assembly and inserting a used needle assembly. The opening 3.1 may be covered by a removable film for maintaining sterility of the needle assembly 2.

Figures 4 and 5 show an exemplary embodiment of a bushing 8 which may be coupled to an inner surface of the proximal portion 7.1 of the housing 7. The bushing 8 may be coupled to the proximal portion 7.1 by an adhesive, friction fit, welding, etc. In another exemplary embodiment, the bushing 8 may replace the proximal portion 7.1 and be a part of the housing 7, integrally formed with the middle portion 7.2.

In an exemplary embodiment, the bushing 8 has a cylindrical shape with a diameter substantially equal to an inner diameter of the proximal portion 7.1 and a height substantially equal to a height of the needle support 5. A wall 8.1 of the bushing 8 has an inner surface 8.2 which may have one or more slots formed therein for interacting with the one or more hooks 5.4. The slot may comprise a first recess 8.3 formed in a distal portion of the bushing 8, a second recess 8.4 adjacent the first recess 8.3, a third recess 8.5 formed in a proximal portion of the bushing 8. Those of skill in the art will understand that the number of slots may be equal to the number of hooks 5.4 formed on the needle support 5. In another exemplary embodiment, the number of slots may be greater than the number of hooks 5.4.

As shown in Figure 5, a first interface 8.6 is established between the first and second recesses 8.3, 8.4, and a second interface 8.7 is established between the second and third recesses 8.4, 8.5. As explained in further detail below, the recesses and interfaces are sized to allow the hook 5.4 to pass therebetween in a predefined sequence.

In an exemplary embodiment, the needle assembly storage system 1 prevents reuse of a used needle assembly. In an initial state, the needle assembly 2 is contained in the storage device 3, and the needle assembly 2 is positioned such that the hook 5.4 resides in the second recess 8.4. The second recess 8.4 is bordered on a first lateral side by a third abutment face 8.10 and on a second lateral side by the second interface 8.7. In an exemplary embodiment, the second recess 8.4 is formed at such a depth in the wall 8.1 that the hook 5.4 is forced substantially into its slot.

When in the injection device is inserted into the needle assembly 2, and the user attempts to secure the needle assembly 2 to the injection device, a torque applied to the needle assembly 2 may attempt to rotate the needle assembly 2 in a first rotational direction A (e.g., clockwise). However, due to the partially extended hook 5.4 abutting the third abutment face 8.10, the needle assembly 2 may not rotate relative to the bushing 8.

When the needle assembly 2 has been secured to the injection device, the needle assembly 2 is withdrawn proximally from the storage device 3. During withdrawal from the storage device 3, the hook 5.4 extends radially as it passes from the second recess 8.4 through the first interface 8.6 to the third recess 8.5, because the third recess is formed a depth in the wall 8.1 greater than the depth of the second interface and the first interface 8.6 is an angled surface formed in the wall 8.1 which connects the second and third recesses 8.4, 8.5. When the needle assembly 2 is fully removed from the bushing 8, the hook 5.4 extends to its fully radially biased position.

After the needle assembly 2 has been used, the needle assembly 2 is returned to the storage device 3. In an exemplary embodiment, a proximal end of the third recess 8.5 may be larger than a width of the hook 5.4. Thus, when the needle assembly 2 is being returned to the storage device 3, the wider proximal end may ensure that the hook 5.4 aligns with the slot. As the needle assembly 2 is inserted into the storage device, the hook 5.4 enters the third recess 8.5 and becomes compressed (radially inward) by the second interface 8.7 and the second recess 8.4. When the needle assembly 2 is inserted into the bushing 8, the injection device may be rotated in a second rotational direction B to disengage the needle assembly 2 therefrom. As torque is applied in the second rotational direction B, the needle assembly 2 may rotate relative to the bushing 8, and the hook 5.4 may compress (radially inward) as it travels over the first interface 8.7 and into the first recess 8.3. The first recess 8.3 may be at a depth in the wall 8.1 greater than the second recess 8.4, such that lateral sides of the first recess 8.3 form a first abutment surface 8.8 and a second abutment surface 8.9, which prevent rotation of the needle assembly 2 relative to the bushing 8. Further, a proximal wall of the first recess 8.3 prevents proximal displacement of the needle assembly 2 relative to the bushing 8. Thus, after the hook 5.4 has traversed the first interface 8.7, it will expand radially outward in the first recess 8.3. An audible feedback (a "click" or "snap" sound) may be provided when the hook 5.4 contacts the wall 8.1 in the first recess 8.3 or when the hook 5.4 passes over the first abutment surface 8.8.

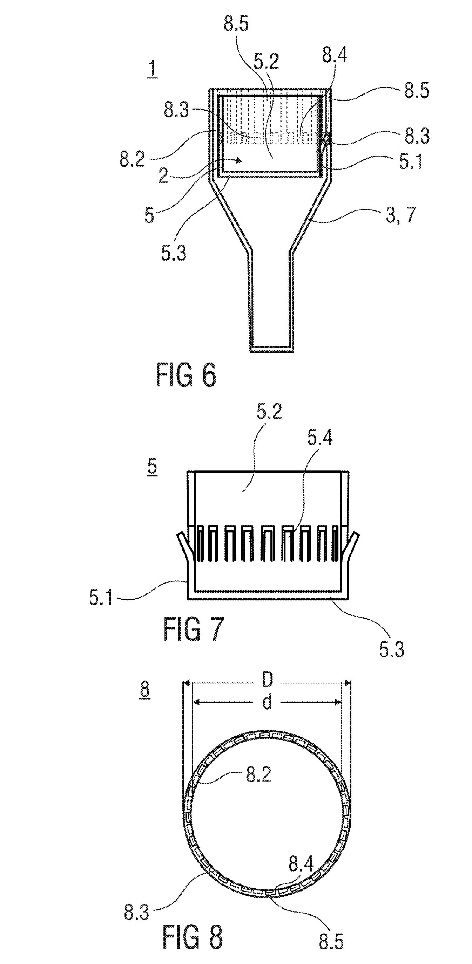

Figure 6 shows another exemplary embodiment of a needle assembly storage system 1 according to the present invention. In this exemplary embodiment, the recesses 8.3, 8.4, 8.5, interfaces 8.6, 8.7 and abutment faces 8.8, 8.9, 8.10 are formed on the proximal part 7.1 of the housing 7.

Figure 7 shows an exemplary embodiment of a needle support 5 of according to the present invention. In this exemplary embodiment, the needle support 5 more than two hooks 5.4 formed on the outer surface 5.1.

Figure 8 shows an exemplary embodiment of a bushing 8 according to the present invention. In this exemplary embodiment, the bushing 8 has more than two recesses 8.3, 8.4, 8.5, interfaces 8.6, 8.7 and abutment faces 8.8, 8.9, 8.10. This exemplary embodiment of the bushing 8 also shows an outer diameter D of the bushing 8 and an inner diameter d of the bushing 8. The outer diameter D may be substantially equal to an inner diameter of the housing 7, and the inner diameter d may be substantially equal to an outer diameter of the needle support 5.1.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

List of References
- 1: needle assembly storage system
- 2: needle assembly
- 3: case
- 3.1: opening
- 4: cavity
- 5: needle support
- 5.1: outer surface
- 5.2: proximal opening
- 5.3: cover
- 5.4: retention hook
- 6: needle
- 6.1: first needle tip
- 6.2: second needle tip
- 7: housing
- 7.1: proximal portion
- 7.2: middle portion
- 7.3: distal portion
- 7.4: distal end
- 8: bushing
- 8.1: wall
- 8.2: inner surface
- 8.3: first recess
- 8.4: second recess
- 8.5: third recess
- 8.6: first interface
- 8.7: second interface
- 8.8: first abutment face
- 8.9: second abutment face
- 8.10: third abutment face
- z: axis
- D: outer diameter of a bushing
- d: inner diameter of a bushing
- A: first rotational direction
- B: second rotational direction

## Claims

1. A needle assembly storage system (1) comprising:
a needle assembly (2) including at least one hook (5.4) formed on an outer surface (5.1); and
a needle assembly storage device (3) having at least one slot for receiving the at least one hook (5.4), the at least one slot including a first recess (8.3) formed in a wall (8.1) of the storage device (3), the first recess (8.3) including a first abutment surface (8.8) and a second abutment surface (8.9) for preventing the at least one hook (5.4) from moving out of the first recess (8.3).

2. The needle assembly storage system (1) according to claim 1, wherein the at least one hook (5.4) has a first state biased in a position extending beyond a circumference of the outer surface (5.1) and a second state in alignment with the circumference of the outer surface (5.1).

3. The needle assembly storage system (1) according to claim 1, wherein an audible feedback is provided when the at least one hook (5.4) engages the first recess (8.3).

4. The needle assembly storage system (1) according to any one of the preceding claims, wherein the at least one slot is formed on a bushing (8) removably coupled to the needle assembly storage device (3).

5. The needle assembly storage system (1) according to any one of the preceding claims, wherein the at least one slot further includes a second recess (8.4) formed in the wall (8.1) adjacent the first recess (8.3), wherein a first depth of the first recess (8.3) in the wall (8.1) is greater than a second depth of the second recess (8.4).

6. The needle assembly storage system (1) according to claim 5, wherein the first recess (8.3) is coupled to the second recess (8.4) via a first interface (8.6), wherein the first interface (8.6) is an angled surface connecting the first abutment surface (8.8) to the second depth.

7. The needle assembly storage system (1) according to claim 5, wherein the at least one slot further includes a third recess (8.5) formed in the wall (8.1) in substantial alignment with the second recess (8.4), wherein a third depth of the third recess (8.5) in the wall (8.1) is greater than the second depth of the second recess (8.4).

8. The needle assembly storage system (1) according to claim 7, wherein the third recess (8.5) is coupled to the second recess (8.4) via a second interface (8.7), wherein the second interface (8.7) is an angled surface connecting the second depth to the third depth.

9. The needle assembly storage system (1) according to claim 7, wherein the third recess (8.5) includes a proximal end having a width greater than a width of one of the at least one hook (5.4).

10. The needle assembly storage system (1) according to claim 1, wherein, when the at least one hook (5.4) is in the first recess (8.3), the first abutment surface (8.8) prevents rotation of the at least one hook (5.4) in a first rotational direction A and the second abutment surface (8.9) prevents rotation of the at least one hook (5.4) in a second rotational direction B.

11. The needle assembly storage system (1) according to any one of the preceding claims, wherein the first recess (8.3) includes a proximal wall to abut the at least one hook (5.4) and prevent proximal displacement of the needle assembly (2) relative to the needle assembly storage device (3).
